# EUROPEAN PATENT SPECIFICATION

(11) **EP 1 554 582 B1**
(45) Date of publication and mention of the grant of the patent: **12.03.2008**
(21) Application number: 03778282.8
(22) Date of filing: 21.10.2003
(51) Int. Cl.: G01N 33/569, G01N 33/576

(54) **METHOD AND KIT FOR DETECTING ANTIGENS**
VERFAHREN UND KIT ZUM NACHWEIS VON ANTIGENEN
PROCEDE ET KIT DE DETECTION D'ANTIGENES

(30) Priority: 23.10.2002 GB 0224688
(43) Date of publication of application: 20.07.2005
(73) Proprietor: GlaxoSmithKline Biologicals s.a., 1330 Rixensart (BE)
(72) Inventor: HVALA, Katia, Laure GlaxoSmithKline, B-1330 Rixensart (BE); LABBE, Dominique, Jean, Germain, B-1330 Rixensart (BE)
(74) Representative: Elmhirst, Elizabeth Lucy
(86) International application number: PCT/EP2003/011810
(87) International publication number: WO 2004/038417

(56) References cited:
- EP-A- 0 294 071
- EP-A- 0 339 667
- YAMAMOTO H ET AL: "QUANTITATION OF GROUP-SPECIFIC A ANTIGEN IN HEPATITIS B VACCINES BYANTI-HBS/A MONOCLONAL ANTIBODY" BIOLOGICALS, ACADEMIC PRESS LTD., LONDON, GB, vol. 25, no. 4, 1997, pages 373-380, XP001009907 ISSN: 1045-1056
- KATZ J B ET AL: "IN VITRO ASSESSMENT OF VIRAL ANTIGEN CONTENT IN INACTIVATED ALUMINUM HYDROXIDE ADJUVANTED VACCINES" JOURNAL OF VIROLOGICAL METHODS, AMSTERDAM, NL, vol. 25, no. 1, 1989, pages 101-108, XP001009902 ISSN: 0166-0934
- DATABASE WPI Derwent Publications Ltd., London, GB; AN 1993-212602 XP002170319 & SU 1 746 318 A (INST. POLIOMIELITA VIRUSNYKH EN), 7 July 1992 (1992-07-07)

## Description

The present invention relates to methods for the detection of antigens. The detection and/or quantification of certain antigens may be required after the antigen has been formulated in some way with additional components. For example, in the case of certain hepatitis B vaccines, the Hepatitis B surface antigen is formulated with aluminium hydroxide. Such formulation with aluminium hydroxide, however, provides problems for the quantification of the antigen component, as the presence of aluminum hydroxide appears in some way (either directly or indirectly) to interfere with the binding of antigen to antibodies in antibody based detection methods, such as an enzyme-linked immunosorbent assay (ELISA).

Yamamoto H et al (Biologicals (1997) 25, 373-380) discloses the quantitation of Group-specific *a* antigen in hepatitis B vaccines by anti-HBs/*a* monoclonal antibodies. EP-A-0 339 667 discloses a method for determining the HAV antigen titre in a hepatitis A,B-combined vaccine. Katz J B et al (Journal of Virological Methods, 25 (1989) 101-108) discloses an in vitro assessment of viral antigen content in inactivated aluminium hydroxide adjuvanted vaccines.

The present invention relates to a method for the detection of an antigen in a sample, the antigen being in a combination with aluminium hydroxide, the method comprising the steps of:
1 contacting the sample with an immunoglobulin, or fragment thereof, in the context of a solid support and in the presence of a basic buffer, to allow binding of the antigen in the sample to the immunoglobulin or fragment thereof;
2 adding a blocking agent; and
3 detecting the binding of antibody to the antigen,
wherein the steps are performed in that order but not necessarily consecutively.

The present invention is based generally on the ELISA detection methods, in which the binding of an antigen to an antibody in the context of a solid support is then detected by the binding of a second antibody. ELISA methods are well known in the art (see, for example, Belanger et al. Clin.Chim. Acta 48, 1973, pages 15-18). Generally the invention thus relates to a method for the detection of an antigen using an antibody or fragment thereof. Suitably the method is an ELISA assay.

The method of the invention relates to the detection of an antigen in combination with aluminium hydroxide. Preferably an antigen which is in combination with aluminium hydroxide is adsorbed or otherwise directly complexed or associated with aluminium hydroxide. The invention, however, also relates to the detection of an antigen which is not itself directly adsorbed or complexed to aluminium hydroxide, but is in a mixture or composition in which aluminium hydroxide is also present. The aluminium hydroxide may be free or bound to an antigen which is not the same as the antigen to be detected by the assay.

Preferably the antigen is a hepatitis antigen, preferably a hepatitis B antigen, most preferably hepatitis B surface antigen.

Preferably the method of the invention is thus for the detection and/or quantification of a hepatitis B surface antigen adsorbed onto aluminium hydroxide.

The invention also relates to detection and/or quantifiation of a hepatitis B antigen adsorbed or associated with an aluminium salt, preferably aluminium phosphate, in a combination with another antigen adsorbed or associated with aluminium hydroxide. For the avoidance of doubt when one antigen is said to be " in a combination with" another antigen then this simply means that the one antigen is "in the presence of" the other antigen, and does not necessarily imply any direct physical interaction between the two. In particular the detection may be of a hepatitis B antigen in a combination of hepatitis B surface antigen adsorbed on aluminium phosphate with pertactin adsorbed onto aluminium hydroxide. In such a case it will be appreciated that the invention applies also to the detection of the pertactin component.

Also preferred is the detection of a hepatitis B antigen in a combination containing diptheria (D) tetanus (T) and acellular pertussis (Pa) ('DTPa') components, such as the GlaxoSmithKline Pediarix^{™} vaccine.

The use of the invention in the detection and/or quantification of Haemophilus influenzae type B purified polyribosyl-ribitol-phosphate (PRP), which binds strongly to aluminium hydroxide, is also preferred.

Preferably the method of the invention is such that there is no or minimal interference of the aluminium hydroxide on the detection / quantification of the antigen of interest.

In the first stage of the process an antigen is contacted with an immunoglobulin or fragment thereof in the context of a solid support. The contacting of antigen with immunoglobulin suitably occurs when one or other is bound to an appropriate solid support. Preferably the immunoglobulin or fragment thereof is bound to the solid support. The solid support is preferably a plastics solid support, suitably a microtitre plate or other plate appropriate for an ELISA-type analysis. Most preferred is a polystyrene microtiter plate, preferably a 96 well microtiter plate, for example the Nunc Maxisorb^{™} flat bottomed microtitre plate.

Preferably the immunoglobulin or fragment thereof is affixed to the solid support and then this fixed immunoglobulin or fragment thereof is contacted with the antigen.

Most preferably a plastics microtitre plate is coated with a suitable immunoglobulin according to well known methods in the art.

Preferably the immunoglobulin component is an antibody or fragment thereof capable of specific binding to the antigen. Suitable fragments of antibodies which retain specific binding activity for a given antigen are well known in the art and may include antibody Fv regions in the absence of Fc regions or may include suitable single chain immunoglobulins. The term 'antibody' will be used herein to describe all suitable immunoglobulins and fragments thereof which have suitable specific binding for an antigen to allow their use in an ELISA or ELISA type detection system. Preferably the antibody is a polyclonal antibody, most preferably a rabbit polyclonal antibody, with a rabbit antibody against hepatitis B most preferred. Preferably the antibody is an IgG molecule.

The production and characterisation of antibodies for the detection of hepatitis B surface antigen is well known, for example, as described in Wands et al, Gastroenterology 80, 225 - 232, 1981, Drouet et al, Med Lab Science 38, 341 - 348, 1981 and Shih JW-K et al J Virol methods, 1, 257 - 273, 1983.

The mixing of the antigen sample with the antibody is carried out in the presence of a basic buffer, which is any suitable buffer having a pH greater than 7. Preferably the buffer has a pH of greater than 8, more preferably having a pH of greater than 8.5 and most preferably having a pH of substantially 9. Preferably the pH is between 7 and 12, more preferably between 8 and 11, most preferably between 8 and 10. The pH can be adjusted to take account of the specific antigen being tested, to optimise the method - that is, to optimise binding and/or minimise the effect of aluminium hydroxide on the assay. Preferably the buffer contains 1% Tween or functional equivalent thereof. Most preferably the buffer is DEA 0.2M, HCl 0.2M at pH9 with 1% Tween added, preferably for use in the detection of hepatitis B surface antigen.

Suitably the antigen to be tested is mixed or diluted into a basic buffer and then contacted with an antibody affixed to a solid support.

Preferably the incubation of the antigen and antibody is carried out with agitation.

Antigen - antibody binding is followed by treatment of the antigen-antibody combination on the solid support with a blocking agent. The blocking agent is any suitable agent that minimise non-specific interactions between the antibody- antigen complex and any detection system used to detect the antibody - antigen complex on the solid support. Suitable blocking agents are well known in the art, such as Fetal calf serum (FCS) or bovine serum albumin (BSA), with a preferred blocking agent being PBS containing 1% BSA.

The detection of the antigen-antibody combination may be carried out using any suitable detection means, and these are well known in the art for the ELISA method. An example of a suitable method is given in Example 1.

Preferably the antibody- antigen combination, after blocking, is contacted with a second antibody which binds specifically to the antigen. The binding of the second antibody may be detected directly or indirectly, suitably in order to assess the quantity of antigen present in the sample. For example, the second antibody may be directly linked to a detectable label or may be detectable by addition of a further labeled antibody. Both direct and indirect detection methods are well known in the art.

Suitable for use in detection is the RF-1 monoclonal antibody (Goodhall AH et al.1981, Medical Laboratory Sciences 38, 349-354), preferably in combination with other monoclonal antibodies. Preferred antibodies are monoclonal antibodies that recognise the epitope of amino acids 111-126 of hepatitis B surface antigen. However, the choice of detection antibody is not critical to the present invention and methods for the production of suitable antibodies are well known in the art.

Preferably the detection of the antibody-antigen complex is carried out in a buffer with a relatively high concentration of protein, such as BSA, to again minimise non-specific interactions. Preferably the detection is carried out in a buffer with at least 0.05% blocking protein, such as BSA, more preferably between 0.05% - 0.5% blocking protein, more preferably between 0.1 - 0.3% blocking protein and most preferably approximately 0.2% blocking protein. Most preferably the detection buffer comprises PBS, 0.2% BSA, 0.1% Tween and 4% newborn calf serum, suitably for use in an assay for hepatitis B surface antigen.

In a most preferred embodiment the present invention relates to a method for the detection of a hepatitis B surface antigen in a sample, the hepatitis antigen being adsorbed onto aluminium hydroxide, the method comprising the steps, in order, of:
1 contacting an antibody specific for hepatitis B surface antigen with a sample to be tested, the antibody being bound to a solid support, the contacting being carried out in the presence of a basic buffer, with agitation, to allow binding of the antigen to the antibody, wherein the buffer has a pH of 9 or approximately pH 9;
2 adding a blocking agent comprising 1% BSA or approximately 1% BSA; and
3 detecting the binding of antibody to antigen.

Preferably the solid support is a microtitre dish.

The method of the present invention is suitably used for quality control purposes for vaccine production. In addition, the method may be generally used for antigen identification, measurement of antigenicity and quantification.

The present invention is hereby illustrated by the following examples that are not binding upon the present invention.

### Example 1 - Detection of hepatitis B Surface antigen adsorbed onto Aluminium hydroxide in a sample

1 Microtiter plates were coated with an anti-HBs rabbit polyclonal antiserum.
2 HBs/Al(OH)₃ samples to be quantified were diluted in DEA 0.2M, HCl 0.2M 1% Tween pH9 (two-fold dilution) and incubated with the plates for 2 hours at 37°C with agitation. Samples tested were Engerix^{™} (hepatitis B) vaccines from GlaxoSmithKline.
3 After a washing step (in 150 mM NaCl, 0.05% Tween) the solid phase was blocked with PBS 1% BSA buffer for 1 hour at 37°C, then washed with NaCl 150 mM + tween 20 0.05% solution.
4 The detection of anti-HBs/HBsAg complex was then performed by addition of a pool of 3 anti-HBs mouse monoclonal antibodies diluted at 1µg/ml in PBS, 0.2% BSA, 0.1% Tween and 4% newborn calf serum and then incubated for 1 hour at 37°C. Excess antibodies were removed by washing and then plates were incubated for 30 min at room temperature (RT) with agitation with a biotin-conjugated anti-mouse Ig (from Prosan™). After washing, the Amdex^{™} streptavidin horseradish peroxydase complex (from Amersham^{™}) was added to the wells (30min at RT with agitation) . Plates were then washed and incubated for 20 min with agitation with a solution of o-phenylenediamine (Sigma^{™}) 0.04%, H₂O₂ 0.03%, 0.1% tween 20, 0.05M citrate buffer pH4.5. The reaction was stopped with H₂SO₄ 2N and read at 490 and 630 nm. The signal obtained at 630nm is subtracted from that at 490nm and can be used to calculate HBsAg concentrations in the sample through reference to a standard by SoftmaxPro (concentration expressed in µg/ml).

The above steps are individually and separately preferred in the present invention, suitably for the detection of hepatitis B surface antigen. Each specific step may be incorporated into the general method of the invention separably from the other steps.

The results obtained were compared that those obtained on Engerix^{™} samples using the commercially available Auszyme^{™} kit (Abbott, Delkenheim, Germany), the currently preferred commercially available kit. The method of the invention gives essentially the same result as that of the Auszyme^{™} kit. The coefficient of variation (CV) is less than 10% indicating high reproducibility of the method.

| | Abbott Auszyme kit | Method of invention |
|---|---|---|
| Recovery | 25.2 µg | 24.7µg |
| CV intra* | 7.7% | 5.9% |
| CV inter** | 6.4% | 8.6% |

| | | |
|---|---|---|
| * variation between the same samples tested on the same day ** variation between different samples tested on different days | | |

### Example 2 - detection of hepatitis surface antigen in a combination vaccine (Pediarix^{™})

Pediarix^{™} comprises hepatitis B surface antigen, DTPa and IPV. The detection of hepatitis B in Pediarix^{™} samples was carried out as follows:
- Coating: Microtiter plates were coated with an anti-HBs rabbit polyclonal antiserum (diluted 1/8000) O/N at 4°C.
- Samples: samples to be quantified were diluted in DEA 0.2M, HCl 0.2M 1 % Tween pH9 (two-fold dilution) and incubated with the plates for 2 hours at 37°C with agitation.
- Blocking was carried out with PBS 1% BSA for 1H at 37°C
- The detection of anti-HBs/HBsAg complex was then performed by addition of a pool of 3 anti-HBs mouse monoclonal antibodies diluted at 1µg/ml in PBS, 1% BSA, 0.1% Tween 20 and 4% newborn calf serum and then incubated for 1 hour at 37°C. Excess antibodies were removed by washing and then plates were incubated for 30 min at room temperature (RT) with agitation with a biotin-conjugated anti-mouse Ig (from Prosan^{™}) diluted in PBS 1% BSA 0.1% Tween 20. After washing, the Amdex^{™} streptavidin horseradish peroxydase complex (from Amersham^{™}) was added to the wells diluted in PBS 1% BSA 0.1% Tween 20(30min at RT with agitation) . Plates were then washed and incubated for 20 min with agitation with a solution of o-phenylenediamine (Sigma^{™}) 0.04%, H₂O₂ 0.03%, 0.1% tween 20, 0.05M citrate buffer pH4.5. The reaction was stopped with H₂SO₄ 2N and read at 490 and 630 nm. HBsAg concentrations in samples were calculated from a reference by SoftmaxPro and expressed in µg/ml.

The above steps are individually and separately preferred in the present invention, suitably for the detection of hepatitis B surface antigen. Each specific step may be incorporated into the general method of the invention separably from the other steps.

The results obtained on Pediarix samples were again consistent with results obtained using the Auszyme kit.

## Claims

1. A method for the detection of an antigen, the antigen being in a combination with aluminium hydroxide, the method comprising the steps of:
(i) contacting the antigen with an immunoglobulin, or fragment thereof, in the context of a solid support and in the presence of a basic buffer, to allow binding of the antigen to immunoglobulin or fragment thereof;
(ii) adding a blocking agent; and
(iii) detecting the binding of antibody to the antigen,
wherein steps 1, 2 and 3 are carried out sequentially but not necessarily consecutively.

2. A method according to claim 1 wherein the antigen is hepatitis B surface antigen.

3. A method according to claim 1 or 2 wherein step (i) is carried out with agitation.

4. A method according to any preceding claim wherein the detection step (iii) is carried out in the presence of 0.2 % bovine serum albumin (BSA).

5. A method according to any preceding claim comprising the following steps:
(i) contacting an antibody specific for hepatitis B surface antigen with a sample to be tested, the antibody being bound to a solid support, the contacting being carried out in the presence of a basic buffer, with agitation, to allow binding of the antigen to the antibody, wherein the buffer has a pH between 8 and 10;
(ii) adding a blocking agent comprising 1% BSA; and
(iii) detecting the binding of antibody to antigen in the presence of 0.2% BSA.

6. A method according to any preceding claim, wherein the basic buffer is pH9.

## Patentansprüche

1. Verfahren zur Detektion eines Antigens, wobei das Antigen in einer Kombination mit Aluminiumhydroxid vorliegt, wobei das Verfahren die folgenden Schritte umfaßt:
(i) Kontaktieren des Antigens mit einem Immunglobulin oder Fragment davon im Kontext eines festen Trägers und in Gegenwart eines basischen Puffers, um das Binden des Antigens an das Immunglobulin oder Fragment davon zu ermöglichen;
(ii) Zugeben eines blockierenden Mittels; und
(iii) Detektieren der Bindung eines Antikörpers an das Antigen,
worin die Schritte 1, 2 und 3 sequentiell durchgeführt werden, jedoch nicht notwendigerweise aufeinander folgend.

2. Verfahren gemäß Anspruch 1, worin das Antigen das Hepatitis B-Oberflächenantigen ist.

3. Verfahren gemäß Anspruch 1 oder 2, worin Schritt (i) unter Bewegung durchgeführt wird.

4. Verfahren gemäß einem der vorangegangenen Ansprüche, worin der Detektionsschritt (iii) in Gegenwart von 0,2 % bovinem Serumalbumin (BSA) durchgeführt wird.

5. Verfahren gemäß einem der vorangegangenen Ansprüche, das die folgenden Schritte umfaßt:
(i) Kontaktieren eines für das Hepatitis B-Oberflächenantigen spezifischen Antikörpers mit einer zu testenden Probe, wobei der Antikörper an einen festen Träger gebunden vorliegt, wobei das Kontaktieren in Gegenwart eines basischen Puffers mit Bewegung durchgeführt wird, um das Binden des Antigens an den Antikörper zu ermöglichen, worin der Puffer einen pH zwischen 8 und 10 hat;
(ii) Zugeben eines blockierenden Mittels, das 1 % BSA umfaßt; und
(iii) Detektieren der Bindung des Antikörpers an das Antigen in Gegenwart von 0,2 % BSA.

6. Verfahren gemäß einem der vorangegangenen Ansprüche, worin der basische Puffer pH 9 aufweist.

## Revendications

1. Procédé de détection d'un antigène, l'antigène étant dans une combinaison avec de l'hydroxyde d'aluminium, le procédé comprenant les étapes consistant à :
(i) mettre en contact l'antigène avec une immunoglobuline, ou un fragment de celle-ci, dans la condition d'un support solide et en présence d'un tampon basique, pour permettre la liaison de l'antigène à l'immunoglobuline ou un fragment de celle-ci ;
(ii) ajouter un agent bloquant ; et
(iii) détecter la liaison de l'anticorps à l'antigène,
dans lequel les étapes 1, 2 et 3 sont réalisées séquentiellement mais pas nécessairement consécutivement.

2. Procédé selon la revendication 1, dans lequel l'antigène est l'antigène de surface de l'hépatite B.

3. Procédé selon la revendication 1 ou 2, dans lequel l'étape (i) est réalisée avec agitation.

4. Procédé selon l'une quelconque des revendications précédentes, dans lequel l'étape (iii) est réalisée en présence de 0,2 % de sérumalbumine bovine (BSA).

5. Procédé selon l'une quelconque des revendications précédentes comprenant les étapes suivantes :
(i) la mise en contact d'un anticorps spécifique de l'antigène de surface de l'hépatite B avec un échantillon à tester, l'anticorps étant lié à un support solide, la mise en contact étant réalisée en présence d'un tampon basique, avec agitation, pour permettre la liaison de l'antigène à l'anticorps, où le tampon a un pH entre 8 et 10 ;
(ii) l'addition d'un agent bloquant comprenant 1 % de BSA ; et
(iii) la détection de la liaison de l'anticorps à l'antigène en présence de 0,2 % de BSA.

6. Procédé selon l'une quelconque des revendications précédentes, dans lequel le tampon basique est de pH 9.
